# EUROPÄISCHE PATENTSCHRIFT

(11) **EP 1 466 170 B1**
(45) Veröffentlichungstag und Bekanntmachung des Hinweises auf die Patenterteilung: **22.11.2006**
(21) Anmeldenummer: 02795239.9
(22) Anmeldetag: 19.12.2002
(51) Int. Cl.: G01N 33/03

(54) **MESSANORDNUNG ZUR BESTIMMUNG EINER EIGENSCHAFT EINES FLUIDES**
MEASURING ASSEMBLY FOR DETERMINING A CHARACTERISTIC OF A FLUID
SYSTEME DE MESURE POUR DETERMINER UNE CARACTERISTIQUE D'UN FLUIDE

(30) Priorität: 17.01.2002 DE 10202002
(43) Veröffentlichungstag der Anmeldung: 13.10.2004
(73) Patentinhaber: TESTO GmbH & Co., 79853 Lenzkirch (DE)
(72) Erfinder: MUHL, Mike, 79106 Freiburg (DE); HALL, Jürgen, 79877 Friedensweiler/Rötenbach (DE)
(74) Vertreter: Schmuckermaier, Bernhard
(86) Internationale Anmeldenummer: PCT/EP2002/014585
(87) Internationale Veröffentlichungsnummer: WO 2003/060499

(56) Entgegenhaltungen:
- EP-A- 0 405 587
- DE-A- 10 015 516
- FR-A- 2 792 414
- US-A- 5 818 731
- US-B1- 6 250 152
- PATENT ABSTRACTS OF JAPAN vol. 012, no. 488 (P-803), 20. Dezember 1988 (1988-12-20) & JP 63 200051 A (HORIBA LTD;OTHERS: 01), 18. August 1988 (1988-08-18)

## Beschreibung

Die Erfindung ist in der Messtechnik einsetzbar und zwar insbesondere bei der Messung von Eigenschaften von Fluiden. Besonders bei Ölen, insbesondere bei flüssigen Frittierfetten, lassen sich bestimmte Eigenschaften aufgrund messbarer dielektrischer Eigenschaften mittels der vorliegenden Erfindung bestimmen.

Die Erfindung bezieht sich auf eine Messanordnung zur Bestimmung einer Eigenschaft, insbesondere des Alterungszustandes eines Fluides insbesondere eines Frittierfettes, aus einer dielektrischen Eigenschaft des Fluides mit einem ersten Sensor zur Messung einer elektrischen Kapazität und mit einem zweiten Sensor zur Temperaturmessung.

Außerdem bezieht sich die Erfindung auf eine Sensoranordnung zur Messung einer dielektrischen Eigenschaft eines Fluides mit einem in das Fluid eintauchbaren dielektrischen Sensor, der einen als Streufeldkondensator ausgebildeten Messkondensator aufweist.

Es ist bekannt, dass sowohl für Speisezwecke verwendete als auch für industrielle Anwendungen in der Mechanik verwendete Öle einem Alterungsprozess unterliegen, der unter anderem durch den Einfluss erhöhter Temperaturen bestimmt ist. Es finden verschiedene chemische Reaktionen statt, die die Qualität des jeweiligen Öls verändern.

Oft findet eine Begutachtung des Öls zunächst aufgrund des optischen Eindrucks, das heißt der mit der Zeit abnehmenden optischen Transmission oder Verfärbung statt.

Diese Größe stellt jedoch nur einen einzelnen Parameter dar, der zur Beurteilung der Qualität im allgemeinen unzureichend ist.

Beispielsweise lässt Frittierfett schon vor einer sichtbaren Verfärbung anhand anderer Parameter einen Qualitätsverlust erkennen, der dazu führen kann, dass ein Austausch notwendig wird.

Die maßgebliche Qualität eines Öls kann beispielsweise durch chemische Tests, auch in Verbindung mit optischen Tests festgestellt werden.

Seit einiger Zeit ist auch die Möglichkeit bekannt, den Alterungszustand eines Öls anhand der gemessenen Dielektrizitätskonstante zu beurteilen.

Eine Schwierigkeit stellt dabei die zusätzliche Abhängigkeit der Dielektrizitätskonstante von der Temperatur dar. Es kann zur Lösung dieses Problems beispielsweise vorgesehen sein, eine Ölprobe auf eine fest vorgegebene Temperatur zu erhitzen oder abzukühlen, um bei dieser Temperatur eine Dielektrizitätsmessung durchzuführen. Eine derartige Meßmethode ist beispielsweise in der US-Patentschrift US 5818731 als Stand der Technik gewürdigt.

Dort sind außerdem Meßmethoden beschrieben, durch die eine Ölqualität mittels mehrerer gemessener physikalischer Parameter bestimmt werden soll, wie beispielsweise einer dielektrischen Messgröße und der Viskosität des Öls.

Da die Farbe eines Frittierfettes eine der sensitivsten Größen für die Bestimmung der Qualität ist, wird gemäß der US-Patentschrift 5818731 vorgeschlagen, eine Dielektrizitätsmessung mit einer Messung der optischen Transmission in einem bestimmten Wellenlängenbereich zu verbinden, um eine umfassende Bewertung der Ölqualität durchzuführen. Dazu wird eine Ölprobe in einen Messbehälter gefüllt und dort mit Licht aus einer Laserdiode mit Licht der Wellenlänge 675 Nanometer bestrahlt, um die Transmission in diesem Wellenlängenbereich zu messen. Außerdem wird mittels eines Messkondensators die Dielektrizitätskonstante gemessen. Die Messung findet dort statt, nachdem die Probe auf eine Temperatur zwischen 155 Grad Celsius und 185 Grad Celsius aufgeheizt worden ist. Nachdem die Dielektrizitätsmessung durchgeführt worden ist, wird mit Hilfe einer Temperaturmessung und einer gespeicherten Regressionskurve die gemessene Dielektrizitätskonstante aufgrund der bekannten Temperaturabhängigkeit auf den Wert bei einer Standardtemperatur zwischen 155 Grad Celsius und 185 Grad Celsius umgerechnet. Dieser Wert soll dann in Verbindung mit der gemessenen Transmission eine Aussage über die Qualität des Fettes erlauben.

Ein Nachteil der bekannten Messanordnungen ist, dass die Messdauer mehrere Minuten bis etwa 10 Minuten beträgt und dass zur Messung eine bestimmte Menge des verwendeten Öles als Probe entnommen und temperiert werden muss. Vor einer neuen Messung muss der Probenbehälter gründlich gereinigt werden.

Die deutsche Patentanmeldung DE 100 15 516 A1 beschreibt ein verfahren zur Bestimmung des Alterungszustands eines Fluids, insbesondere eines Frittierfettes, aus einer dielektrischen Eigenschaft des Fluides. Dessen Dielektrizitätskonstante wird mit einem Messkopf, an dem ein Sensor angebracht ist, gemessen. Der Messwert wird in der Geräteelektronik weiter verarbeitet und eine Aussage über den Qualitätszustand des Öles gemacht.

Der vorliegenden Erfindung liegt demgegenüber die Aufgabe zugrunde, eine Messanordnung und eine Sensoranordnung der eingangs genannten Art zu schaffen, die einfach aufgebaut sind und mit geringem konstruktiven Aufwand sowie bei einfacher und wenig aufwendiger Bedienung eine schnelle Bestimmung der Qualität des jeweiligen Fluides erlauben.

Die Aufgabe wird durch eine Messanordnung gemäß Patentanspruch 1 gelöst. Vorteilhafte Ausgestaltungen sind Gegenstand der Unteransprüche.

Dadurch, dass die Dielektrizitätskonstante und die Temperatur gemessen wird, ist grundsätzlich eine Aussage über den Zustand und die Eigenschaften des jeweiligen Fluids ableitbar. Die Ausführung der Sensoren als eintauchbare Sensoren erlaubt die Messung in situ, ohne dass eine Probe zur Einfüllung in eine Messanordnung entnommen werden muss. Dadurch wird die Messzeit verkürzt einerseits hinsichtlich der entfallenden Notwendigkeit der Probenentnahme, andererseits dadurch, dass bei der in dem Fluidvolumen gegebenen Temperatur ohne eine Temperaturänderung gemessen wird. Dadurch wird zwar die Auswertung der Messwerte anspruchsvoller, jedoch wird der Messvorgang selbst einfacher und schneller ausführbar. Bei der Auswertung muss die jeweilige Temperatur, bei der gemessen wird, berücksichtigt werden, wenn die gemessene Dielektrizitätskonstante zur Ableitung einer Qualitätsaussage über das Fluid ausgewertet wird.

Eine derartige Messanordnung ist leicht transportabel und kann zur Messung in Behältern, in denen das Fluid verwendet wird beispielsweise in Friteusen, direkt verwendet werden. Derartige Messanordnungen können auch dauerhaft in Friteusen zur Überprüfung des verwendeten Frittierfettes eingebaut werden beziehungsweise nachgerüstet werden.

Eine vorteilhafte Ausgestaltung der Erfindung sieht vor, dass der erste und der zweite Sensor mit einer Auswerteeinrichtung verbunden sind, die jeweils einem gemessenen Temperaturwert und einem gemessenen elektrischen Kapazitätswert einen Wert der zu bestimmenden Eigenschaft zuordnet.

In der Auswerteeinrichtung kann entweder ein Rechnenalgorithmus oder eine Wertematrix hinterlegt sein, mittels deren dem jeweils gemessen Temperaturwert und dem gemessenen Kapazitätswert beziehungsweise der daraus sich ergebenden Dielektrizitätskonstante ein Qualitätswert des Fluides, beispielsweise ein Alterungszustand eines Frittierfettes zugeordnet wird. Beispielsweise kann mittels vorher empirisch ermittelter Werte aus der Dielektrizitätskonstanten und der Temperatur, bei der diese gemessen wurde, eine Konzentration von bestimmten polaren Stoffanteilen in dem Fluid bestimmt werden, die ihrerseits auf den Alterungszustand des Fluids schließen lassen.

Eine weitere vorteilhafte Ausgestaltung der Erfindung sieht vor, dass in einer vergleichseinrichtung der Auswerteeinrichtung der jeweils durch den dielektrischen Sensor gemessene Wert der elektrischen Kapazität mit einem dem gemessenen Temperaturwert zugeordneten gespeicherten Referenzwert verglichen und in Abhängigkeit vom Erreichen oder Überschreiten des Referenzwertes ein Signal ausgegeben wird.

In diesem Fall enthält die Auswerteeinrichtung für jeden Temperaturwert, bei dem eine Messung durchgeführt werden kann, also beispielsweise zwischen 30 Grad Celsius und 200 Grad Celsius, in Abständen von 0,5 Grad Celsius oder 1 Grad Celsius einen Wert der gemessenen Kapazität beziehungsweise der daraus ermittelten Dielektrizitätskonstante, der bezüglich des sich ergebenden Alterungszustandes des Fluides gerade noch vertretbar ist. Wird dieser Wert als Referenzwert überschritten, so gibt die Auswerteeinrichtung nach einem Vergleich ein Signal aus, das den Benutzer warnt, beispielsweise in Form eines optischen oder akustischen Warnsignals.

Wesentliches Merkmal der Erfindung ist eine Kompensationseinrichtung zur Korrektur des Messwertes der elektrischen Kapazität unter Berücksichtigung eines an einem in der Nähe des Messkondensators angeordneten Hilfskondensator gemessenen Referenzmesswertes einer Kapazität.

Die Dielektrizitätskonstante des Fluids wird dadurch bestimmt, dass der Einfluss des Fluids auf den als Messkondensator dienenden Streufeldkondensator bestimmt wird, wenn dieser in das Fluid eingetaucht ist. Durch eine hohe Dielektrizitätskonstante des Fluids ergibt sich eine Vergrößerung der elektrischen Kapazität des Messkondensators. Dabei ist jedoch zu berücksichtigen, dass auch über das Eintauchen des Messkondensators in das Fluid hinaus störende Einflüsse bei der Messung der Kapazität auftreten können. Beispielsweise besteht auch zwischen den Zuleitungen des Messkondensators eine Kapazität, die durch äußere Einflüsse geändert werden kann. Tauchen die Zuleitungen des Messkondensators in das Fluid ein, so erhöht sich auch zwischen ihnen die Kapazität, was zu einer Störung der durchzuführenden Kapazitätsmessung an dem Messkondensator selbst führt. Aus diesem Grund ist ein Hilfskondensator vorgesehen, dessen Kapazität sich beispielsweise bei einem zu weiten Eintauchen des Messkondensators in das Fluid in dem selben Sinn ändert, wie die Kapazität der Zuleitungen des Messkondensators. Wird die Kapazität des Hilfskondensators überwacht, so ergibt eine Erhöhung der elektrischen Kapazität des Hilfskondensators, dass dieser in das Fluid eingetaucht ist. Dies führt zu einer notwendigen Kompensation der Messung am Messkondensator. Die dort gemessene Kapazität ist durch die Einwirkungen auf die Zuleitung verfälscht und muss entsprechend kompensiert werden.

Die Kompensation kann auch vorsehen, dass bei der Beobachtung einer Erhöhung der Kapazität des Hilfskondensators der Sensor aus dem Fluid ein Stück weit herausgezogen wird, bis die Kapazität des Hilfskondensators dem Normalwert entspricht. Dann ist sichergestellt, dass auch die Zuleitungen des Messkondensators nicht in das Fluid hineinragen.

Aber auch andere Umgebungseinflüsse auf die Zuleitungen des Messkondensators beziehungsweise auf den Messkondensator selbst, beispielsweise Temperatureinflüsse, die über die Abhängigkeit der Dielektrizitätskonstanten des Fluids von der Temperatur hinausgehen, können durch die Einbeziehung des Verhaltens des Hilfskondensators kompensiert werden.

Die erfindungsgemäße Messanordnung sieht außerdem vor, dass bei einer sensoranordnung zur Messung einer dielektrischen Eigenschaft eines Fluides mit einem in das Fluid eintauchbaren dielektrischen Sensor, der einen als Streufeldkondensator ausgebildeten Messkondensator aufweist, der Sensor einen Hilfskondensator aufweist und dass beim Einbringen des Sensors in das Fluid der Hilfskondensator frühestens dann in das Fluid eintaucht, wenn der Messkondensator in das Fluid völlig eingetaucht ist.

Eine derartige Sensoranordnung ist in dem oben geschilderten Sinne optimal für die Messanordnung die ebenfalls Gegenstand der vorliegenden Erfindung ist, zu verwenden. Es kann dann für eine möglichst gute Kompensation vorgesehen sein, dass Zuleitungen des Messkondensators und des Hilfskondensators symmetrisch und baugleich zueinander ausgebildet sind. Sie unterliegen dann denselben Störungseinflüssen in derselben Weise.
Außerdem kann die erfindungsgemäße Sensoranordnung so ausgestaltet sein, dass der Hilfskondensator aus zwei vor dem Messkondensator endenden Stichleitungen besteht, die gleichartig wie die Zuleitungen des Messkondensators ausgebildet und angeordnet sind. Durch die Symmetrie der zuleitungen des Messkondensators und des Hilfskondensators können eventuelle Störeinflüsse, die auf beide Zuleitungen gleichmäßig wirken, optimal kompensiert werden, beispielsweise durch Subtraktion der Messwerte.

Eine vorteilhafte Ausgestaltung der erfindungsgemäßen Sensoranordnung sieht vor, dass der Messkondensator durch eine Mehrzahl von flachen Leiterbahnen insbesondere in Form eines Interdigitalkondensators gebildet ist.

Die Sensoranordnung kann beispielsweise dadurch besonders einfach aufgebaut sein, dass die Leiterbahnen in Dünnschicht- oder Dickschichttechnik auf einen isolierenden Träger aufgedruckt sind.

Die Leiterbahnen können beispielsweise auf einen flachen, jedoch auch auf einen runden oder zylindrischen Körper aufgebracht sein.

Die zylindrische Form zeichnet sich dadurch aus, dass sie besonders platzsparend ist, während die flache Bauform durch die größere Interaktionsfläche mit dem Fluid eine sehr geringe Zeit zum Temperaturausgleich in dem Fluid benötigt.

Vorteilhaft kann bei der erfindungsgemäßen Sensoranordnung der Temperatursensor in Form eines NTC-Widerstandes, eines PCT-Widerstandes oder eines Temperaturelementes ausgeführt sein. Diese Temperatursensoren sind kostengünstig, gut eichbar und widerstandsfähig sowie in ihrem Verhalten stabil, so dass die gesamte Sensoranordnung nicht allzu häufig kalibriert werden muss.

Es erweist sich als günstig, wenn der Temperatursensor mit dem dielektrischen Sensor zu einer konstruktiven Einheit verbunden ist. Der Temperatursensor kann beispielsweise an dem Träger für die Leiterbahnen des Messkondensators befestigt sein. In diesem Falle vereinfacht sich die Verwendung der Sensoranordnung beziehungsweise der Messanordnung, da nur eine einzige Sonde mit den beiden Sensoren in das Fluid beziehungsweise in das Frittierfett eingebracht werden muss.

Als vorteilhaft ergibt sich außerdem, dass die Zuleitungen des Temperatursensors auf den isolierenden Träger in Form von Leiterbahnen aufgebracht sind. Durch diese bauliche Ausführung ist die Sensoranordnung besonders einfach und kostengünstig aufgebaut und es ergibt sich durch die Zuleitungen des Temperatursensors auch keine Störung bei den Kapazitätsmessungen.

Im folgenden wird die Erfindung anhand eines Ausführungsbeispiels in einer Zeichnung gezeigt und anschließend beschrieben. Dabei zeigt
Figur 1 schematisch eine erfindungsgemäße Messanordnung in einer ersten Ausführung,
Figur 2 schematisch die erfindungsgemäße Messanordnung in einer zweiten Ausführung,
Figuren 3, 4, 5 verschiedene Ausgestaltungen eines dielektrischen Sensors, in Figur 5 mit einem Temperatursensor.

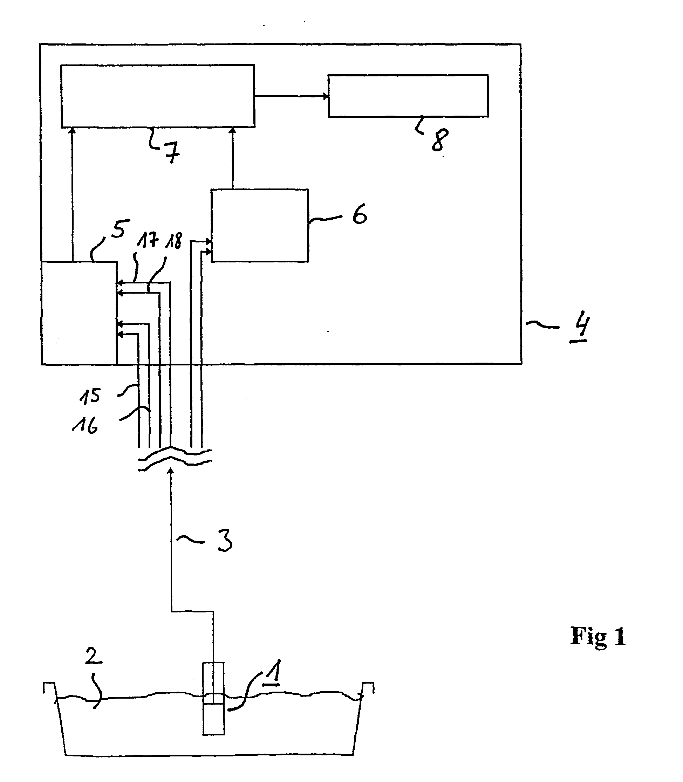

In der Figur 1 ist schematisch eine Messanordnung dargestellt, die eine Sensoranordnung 1 aufweist, die in ein Fluid 2, beispielsweise ein Frittierfett eingetaucht ist. Die Sensoranordnung 1 weist einen dielektrischen Sensor sowie einen Temperatursensor auf, die weiter unten näher beschrieben werden.

Die Sensoranordnung 1 ist über elektrische Zuleitungen 3 mit einer digitalen Auswerteeinrichtung 4 verbunden. Die Auswerteeinrichtung 4 weist eine erste Recheneinrichtung 5 auf, in der aus den Messdaten eine Kapazität, eine Dielektrizitätskonstante oder ein dieser entsprechender Wert bestimmt wird. In einer zweiten Recheneinrichtung 6 wird aus den von dem Temperatursensor gelieferten Daten die Temperatur des Fluids bestimmt.

In einer dritten Recheneinrichtung 7 wird dem Wert der Dielektizitätskonstante und der gemessenen Temperatur ein temperaturunabhängiger Wert der Dielektrizitätskonstante zugeordnet, der ein objektives Kriterium für den Zustand des Fluides, in diesem Fall den Alterungszustand des Frittierfettes darstellt. Dies kann beispielsweise ein auf eine feste Temperatur bezogener Wert sein. Dieser so bestimmte Wert wird in der Anzeige 8 dargestellt und an den Benutzer ausgegeben. Anstelle der Anzeige 8 kann auch ein Interface zur Übergabe der Daten an ein weiteres Datenverarbeitungsgerät vorgesehen sein.

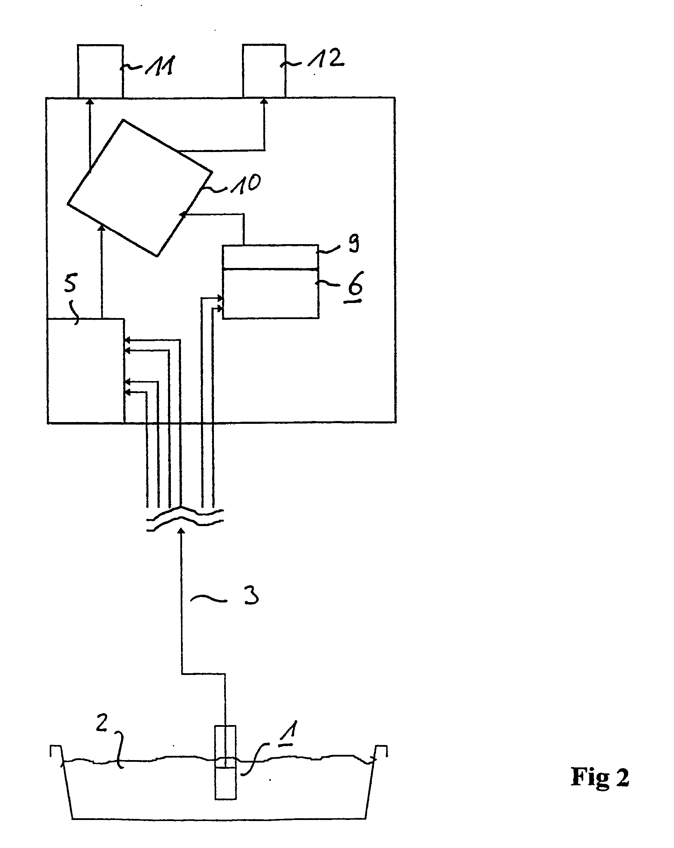

In der Figur 2 ist eine ähnliche Messanordnung wie in Figur 1 dargestellt, wobei gleiche Elemente mit dem selben Bezugszeichen bezeichnet sind wie in der Figur 1.

Ebenso wie in dem oben beschriebenen Beispiel wird in der ersten Recheneinrichtung die Dielektrizitätskonstante oder eine entsprechende Größe bestimmt. In der zweiten Recheneinrichtung 6 wird die Temperatur bestimmt.

Zusätzlich wird in der zweiten Recheneinrichtung 6 der Temperatur aufgrund von gespeicherten Referenzdaten für verschiedene Temperaturwerte eine bestimmte, bei der jeweiligen Temperatur gerade noch zulässige Dielektrizitätskonstante oder ein entsprechender Wert, beispielsweise die gemessene Kapazität für das zu vermessende Frittierfett zugeordnet. Die Referenzdaten sind in einer Speichereinheit 9 gespeichert. Der durch die erste Recheneinrichtung 5 bestimmte Wert der Dielektrizitätskonstante oder der entsprechenden Größe wird mit dem durch die zweite Recheneinrichtung 6 der gemessenen Temperatur zugeordneten gerade noch zulässigen Referenzgröße in der Vergleichseinrichtung 10 verglichen. Der Vergleich wird derart bewertet, dass bei einer Übereinstimmung der beiden Werte oder einer Unterschreitung des Referenzwertes eine entsprechende erste Anzeigeeinrichtung 11 betätigt wird, die anzeigt, dass das Frittierfett noch in Ordnung und verwendbar ist.

Übersteigt die durch die erste Recheneinrichtung 5 bestimmte Dielektrizitätskonstante oder entsprechende Größe den zugeordneten Referenzwert, so wird die zweite Anzeigeeinrichtung 12 bestätigt, durch die angezeigt wird, dass das Frittierfett nicht mehr verwendet, sondern ausgetauscht werden soll. Die erste Anzeigeeinrichtung 11 kann beispielsweise als grüne Leuchte, die zweite Anzeigeeinrichtung 12 als rote Leuchte ausgestaltet sein. Die Vergleichseinrichtung 10 kann auch so eingerichtet sein, dass bereits bei einer Übereinstimmung des gemessenen Wertes für die Dielektrizitätskonstante mit dem Referenzwert das Frittierfett verworfen und ein notwendiger Wechsel mittels der zweiten Anzeigeeinrichtung angezeigt wird.

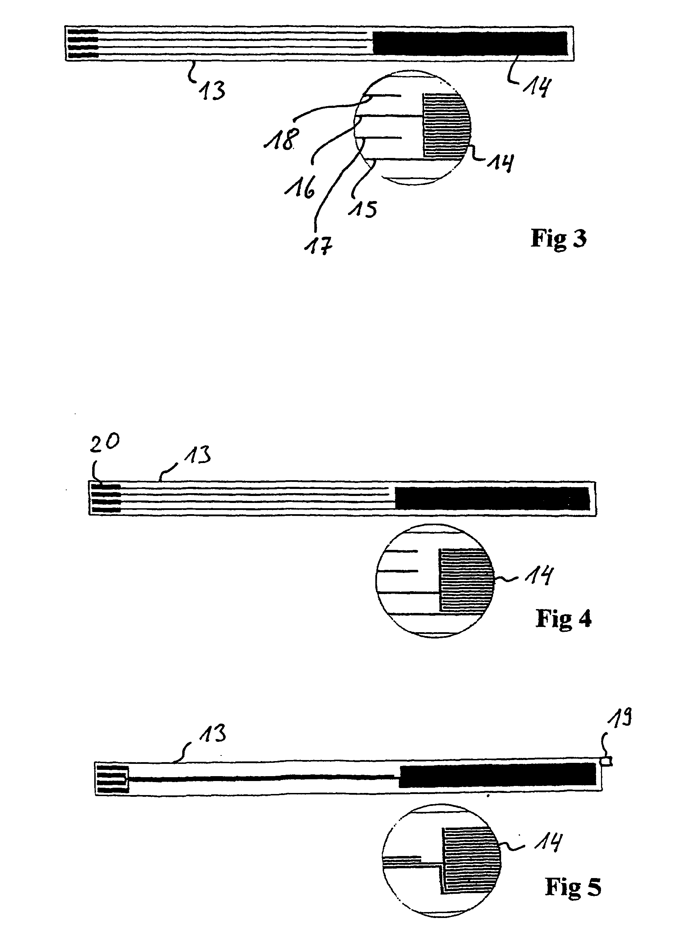

Im folgenden soll genauer die Gewinnung der Messwerte für die Dielektrizitätskonstante und die Temperatur beschrieben werden. Dabei wird zunächst auf die Figuren 3, 4 und 5 bezug genommen. In der Figur 3 ist ein Teil der in den Figuren 1 und 2 dargestellten Sensoranordnung 1 in einer Seitenansicht dargestellt. Die Figur 3 zeigt einen flachen Keramikträger, 13, auf den flache Leiterbahnen mittels Dünnschicht- oder Dickschichttechnik aufgedruckt sind. Die Leiterbahnen bestehen vorteilhaft aus Edelmetall, zum Beispiel Gold. Im linken Teil der Darstellung sind vier Zuleitungen gezeigt, die gleichartig ausgebildet sind und parallel zueinander verlaufen.

Im rechten Teil der Darstellung ist der Messkondensator 14 dargestellt, der in Form eines Streufeldkondensators mit mäanderförmig ineinander verschlungenen Leiterbahnen ausgebildet ist. Der Messkondensator ist in der kreisförmig dargestellten Detailvergrößerung ein zweites Mal gezeigt und dort besser zu erkennen. Er weist zwei Zuleitungen 15, 16 auf, die von dem Messkondensator 14 bis zu der ersten Recheneinrichtung 5 der Auswerteeinrichtung 4 führen.

Die Kapazität des Messkondensators 14 ist, da die Feldlinien dessen unmittelbare Umgebung durchsetzen, von dem Medium abhängig, in dem der Messkondensator 14 sich befindet. Wird der Messkondensator, wie in den Figuren 1 und 2 dargestellt, in ein Fluid 2 eingetaucht, das eine höhere Dielektrizitätskonstante aufweist als Luft, so vergrößert sich die Kapazität des Messkondensators 14 erheblich. Von der Vergrößerung der gemessenen Kapazität kann auf die Dielektrizitätskonstante des den Messkondensator 14 umgebenden Mediums geschlossen werden.

Außer den Zuleitungen 15, 16 des Messkondensators sind auf dem Keramikträger 13 die Zuleitungen 17, 18 vorgesehen, die als blinde Stichleitungen vor dem Messkondensator 14 enden und die einen Hilfskondensator bilden, dessen Kapazität in der Auswerteeinrichtung 4 ebenfalls überwacht wird. Änderungen der Umgebung der Zuleitungen 15, 16 des Messkondensators, die die Kapazitätsmessung an dem Messkondensator 14 verfälschen würden, verändern ebenfalls die Messung des Hilfskondensators, der durch die Zuleitungen 17, 18 gebildet ist. Durch die Änderung der Kapazität des Hilfskondensators kann die Größe der Störung bestimmt und die Störung der Messung an dem Messkondensator kompensiert werden. Dies kann zum Beispiel dann wichtig werden, wenn die Sensoranordnung 1 so tief in das Fluid 2 eingetaucht wird, dass die Zuleitungen 15, 16, 17, 18 zu einem Teil in das Fluid eintauchen und somit die Kapazität der Zuleitungen signifikant erhöht wird.

Durch diese Kompensation, die in einer Kompensationseinrichtung 5a der ersten Recheneinheit 5 geschieht, wird die Messung der Dielektrizitätskonstante des Fluids wesentlich weniger fehleranfällig und unabhängig von einer idealen Handhabung der Sensoranordnung. Auch die Kalibrierung der Messanordnung kann gegenüber bekannten Messanordnungen wesentlich seltener erfolgen.

Während gemäß der Figur 3 die Zuleitungen 15, 16 des Messkondensators und 17, 18 abwechselnd zueinander angeordnet sind, zeigt die Figur 4 eine Anordnung, bei der jeweils die beiden Zuleitungen des Messkondensators 14 und die beiden blinden Stichleitungen des Hilfskondensators direkt nebeneinander angeordnet sind. Dabei ist der Abstand zwischen den Zuleitungen so gewählt, dass die gesamte Breite der nebeneinander und parallel verlaufenden Zuleitungen etwa der Breite des Messkondensators 14 entspricht.

In der Figur 5 ist dagegen eine Anordnung dargestellt, bei der die Zuleitungen des Messkondensators 14 direkt nebeneinander und ebenso die Stichleitungen direkt nebeneinander und parallel zueinander angeordnet sind, wobei jedoch alle vier Leitungen sehr eng parallel zueinander geführt sind, was zu einer Erhöhung der Zuleitungskapazität führt. Dadurch wird die Anordnung beispielsweise sensitiver dafür, ob der Sensor so tief in das Fluid eingetaucht ist, dass auch die Zuleitungen bereits von dem Fluid umgeben sind.

An dem Ende des in der Figur 5 dargestellten Keramikträgers 13 ist beispielhaft und schematisch ein Temperatursensor 19 in Form eines Temperaturelementes dargestellt. Durch dieses wird bei eingetauchtem Messkondensator 14 in dessen unmittelbarer Umgebung die Temperatur des Fluids gemessen. Der Temperatursensor 19 ist mittels zweier Zuleitungen, die auf der Rückseite des Keramikträgers 13 verlaufen und in der Figur nicht dargestellt sind, mit der Auswerteeinrichtung 4 und dort mit der zweiten Recheneinrichtung 6 zur Bestimmung der Temperatur verbunden.

Die in den Figuren 3, 4 und 5 dargestellten Zuleitungen enden am Ende des Keramikträgers 13 jeweils mit verbreiterten Leiterbahnstücken 20, die als Steckkontakte für einen dort aufzusetzenden Stecker dienen können der das Ende eines flexiblen Kabels bildet, dessen anderes Ende mit der Auswerteeinrichtung 4 verbunden ist. Das flexible Kabel kann entsprechend abgeschirmt werden, um Einflüsse auf die Kapazität der Zuleitungen der Kondensatoren zu verhindern.

Durch die dargestellte Messanordnung in Verbindung mit der hierfür verwendeten Sensoranordnung sind kurze Messzeiten, hohe Messgenauigkeiten und ein reduzierter Kalibrieraufwand bei der Bestimmung von dielektrischen Eigenschaften von Ölen, insbesondere Frittierfetten gegeben. Die Messeinrichtung kann jedoch auch bei der Bestimmung anderer, mit der Dielektrizitätskonstanten zusammenhängender Größen von Fluiden dienen. Dies beschränkt sich nicht auf Flüssigkeiten sondern die Anwendung ist beispielsweise auch in Gasen, beispielsweise Isoliergasen für elektrische Anlagen denkbar. Als Massenprodukt ist beispielsweise auch der serienmäßige Einsatz einer Messeinrichtung, wie sie hier dargestellt ist, bei der Überwachung der Qualität von Motoröl von Kraftfahrzeugen denkbar. Die Benachrichitung des Kraftfahrzeugführers wird dann nicht nur in Abhängigkeit von der Motorlaufleistung, sondern nach tatsächlich erfolgter Qualitätsmessung des Motoröls durch eine Leuchte am Armaturenbrett erfolgen können.

## Patentansprüche

1. Messanordnung zur Bestimmung einer Eigenschaft eines Fluides, insbesondere des Alterungszustandes eines Fluides (2), insbesondere eines Frittierfettes, aus einer dielektrischen Eigenschaft des Fluides mit einem ersten Sensor (14) zur Messung einer elektrischen Kapazität und mit einem zweiten Sensor (19) zur Temperaturmessung, wobei
der erste Sensor als ein in das Fluid eintauchbarer, einen als Messkondensator dienenden Streufeldkondensator aufweisender dielektrischer Sensor (14) und der zweite Sensor als in das Fluid (2) eintauchbarer Temperatursensor (19) ausgebildet ist
**dadurch gekennzeichnet, daß**
die Messanordnung einen Hilfskondensator (17, 18) aufweist, dessen Kapazität sich aufgrund äußerer Einflüsse in dem selben Sinn ändert wie die Kapazität von Zuleitungen des Messkondensators, daß eine Kompensationseinrichtung (5a) vorgesehen ist zur Korrektur des Messwertes der elektrischen Kapazität des Messkondensators (14) unter Berücksichtigung eines in dem in der Nähe des Messkondensators (14) angeordneten Hilfskondensator (17, 18) gemessenen Referenzwertes einer Kapazität und daß beim Einbringen des Sensors (14) in das Fluid der Hilfskondensator (17, 18) frühestens dann in das Fluid (2) eintaucht, wenn der Messkondensator in das Fluid (2) völlig eingetaucht ist.

2. Messanordnung nach Anspruch 1,
**dadurch gekennzeichnet, daß**
der erste und der zweite Sensor (14, 19) mit einer Auswerteeinrichtung (4) verbunden sind, die jeweils einem gemessenen Temperaturwert und einem gemessenen elektrischen Kapazitätswert einen Wert der zu bestimmenden Eigenschaft zuordnet.

3. Messanordnung nach Anspruch 1 oder 2,
**dadurch gekennzeichnet, daß**
in einer Vergleichseinrichtung der Auswerteeinrichtung (4) der jeweils durch den dielektrischen Sensor (14) gemessenen Wert der elektrischen Kapazität mit einem dem gemessenen Temperaturwert zugeordneten gespeicherten Referenzwert verglichen und in Abhängigkeit vom Erreichen oder Überschreiten des Referenzwertes ein Signal ausgegeben wird.

4. Messanordnung nach Anspruch 1, 2 oder 3,
**dadurch gekennzeichnet, daß** Zuleitungen (15, 16, 17, 18) des Messkondensators und des Hilfskondensators (17, 18) symmetrisch und baugleich zueinander ausgebildet sind.

5. Messanordnung nach einem der Ansprüche 1 bis 4,
**dadurch gekennzeichnet, daß**,
der Hilfskondensator (17, 18) aus zwei vor dem Messkondensator endenden Stichleitungen besteht, die gleichartig wie die Zuleitungen (15, 16) des Messkondensators ausgebildet und angeordnet sind.

6. Messanordnung nach einem der Ansprüche 1 bis 5,
**dadurch gekennzeichnet, daß**
der Messkondensator durch eine Mehrzahl von flachen Leiterbahnen insbesondere in Form eines Interdigitalkondensators gebildet ist.

7. Messanordnung nach Anspruch 6, **dadurch gekennzeichnet, daß**
die Leiterbahnen in Dünnschicht- oder Dickschichttechnik auf einen isolierenden Träger (13) aufgedruckt sind.

8. Messanordnung nach einem der Ansprüche 1 bis 7,
**gekennzeichnet durch** einen Temperatursensor (19) in Form eines NTC-Widerstandes, eines PTC-Widerstandes oder eines Temperaturelementes.

9. Messanordnung nach Anspruch 8,
**dadurch gekennzeichnet, daß** ,
der Temperatursensor (19) mit dem dielektrischen Sensor (14) zu einer konstruktiven Einheit verbunden ist.

10. Messanordnung nach Anspruch 8 oder 9,
**dadurch gekennzeichnet, daß**,
die Zuleitungen des Temperatursensors auf den isolierenden Träger (13) in Form von Leiterbahnen aufgebracht sind.

## Claims

1. Measurement assembly to determine a characteristic of a fluid, in particular the ageing state of a fluid (2), in particular a deep-frying fat, from a dielectric property of the fluid, with a first sensor (14) to measure an electrical capacitance and with a second sensor (19) for temperature measurement,
wherein the first sensor is formed as a dielectric sensor (14) that can be immersed in the fluid and has a scatter field capacitor serving as a measurement capacitor, and the second sensor is formed as a temperature sensor (19) that can be immersed in the fluid (2),
**characterised in that** the measurement assembly has an auxiliary capacitor (17, 18), the capacitance of which changes on the basis of external influences in the same sense as the capacitance of the supply lines of the measurement capacitor, that a compensation device (5a) is provided to correct the measured value of the electrical capacitance of the measurement condenser (14) taking into account a reference value of a capacitance measured in the auxiliary capacitor (17, 18) arranged in the vicinity of the measurement capacitor (14), and that on introduction of the sensor (14) into the fluid the auxiliary capacitor (17, 18) is immersed in the fluid (2) at the earliest when the measurement capacitor is fully immersed in the fluid (2).

2. Measurement assembly according to claim 1, **characterised in that** the first and second sensors (14, 19) are connected with an analysis device (4) which allocates a value of the characteristic to be determined to a measured temperature value and to a measured electrical capacitance value.

3. Measurement assembly according to claims 1 or 2, **characterised in that** in a comparison device of the analysis device (4), the value of the electrical capacitance measured by the dielectric sensor (14) is compared with a stored reference value allocated to the measured temperature value and a signal is output as a function of reaching or exceeding the reference value.

4. Measurement assembly according to claim 1, 2 or 3, **characterised in that** supply lines (15, 16, 17, 18) of the measurement capacitor and auxiliary capacitor (17, 18) are formed symmetrically and identical to each other in construction.

5. Measurement assembly according to any of claims 1 to 4, **characterised in that** the auxiliary capacitor (17, 18) comprises two stub cables ending in front of the measurement capacitor which are formed and arranged in the same way as the supply lines (15, 16) of the measurement capacitor.

6. Measurement assembly according to any of claims 1 to 5, **characterised in that** the measurement capacitor is formed by a multiplicity of flat conductor tracks in particular in the form of an inter-digital capacitor.

7. Measurement assembly according to claim 6, **characterised in that** the conductor tracks are printed in thin or thick layer technology onto an insulated carrier (13).

8. Measurement assembly according to any of claims 1 to 7, **characterised by** a temperature sensor (19) in the form of an NTC resistor, a PTC resistor or a temperature element.

9. Measurement assembly according to claim 8, **characterised in that** the temperature sensor (19) is connected with the dielectric sensor (14) to form a constructional unit.

10. Measurement assembly according to claim 8 or 9, **characterised in that** the supply lines of the temperature sensor are applied to the insulating carrier (13) in the form of conductor tracks.

## Revendications

1. Système de mesure pour déterminer une caractéristique d'un fluide, et en particulier l'état de vieillissement d'un fluide (2), notamment d'une graisse de friture, à partir d'une caractéristique diélectrique du fluide,
comportant un premier capteur (14) pour mesurer une capacité électrique et un second capteur (19) pour mesurer la température,
le premier capteur ayant la forme d'un capteur diélectrique (14) pouvant être immergé dans le fluide et comportant un condensateur à champ de dispersion servant de condensateur de mesure, et le second capteur ayant la forme d'un capteur de température (19) pouvant être immergé dans le fluide (2),
**caractérisé en ce que**
le système de mesure comporte un condensateur auxiliaire (17, 18) dont la capacité varie en raison d'influences extérieures dans le même sens que la capacité des câbles d'alimentation du condensateur de mesure,
un dispositif de compensation (5a) est prévu pour corriger la valeur mesurée de la capacité électrique du condensateur de mesure (14) compte tenu d'une valeur de référence d'une capacité, mesurée dans le condensateur auxiliaire (17, 18) disposé à proximité du condensateur de mesure (14),
et lorsque l'on introduit le capteur (14) dans le fluide, le condensateur auxiliaire (17, 18) s'immerge dans le fluide (2) au plus tôt au moment où le condensateur de mesure est entièrement immergé dans le fluide (2).

2. Système de mesure selon la revendication 1,
**caractérisé en ce que**
le premier et le second capteur (14, 19) sont reliés à un dispositif d'exploitation (4) qui associe à chaque fois, à une valeur de température mesurée et à une valeur de capacité électrique mesurée, une valeur de la caractéristique à déterminer.

3. Système de mesure selon la revendication 1 ou 2,
**caractérisé en ce que**
dans un dispositif de comparaison du dispositif d'exploitation (4), la valeur de la capacité électrique mesurée à chaque fois au moyen du capteur diélectrique (14) est comparée à une valeur de référence enregistrée, associée à la valeur de température mesurée, et un signal est émis en fonction de l'atteinte ou du dépassement de la valeur de référence.

4. Système de mesure selon la revendication 1, 2 ou 3,
**caractérisé en ce que**
les câbles d'alimentation (15, 16, 17, 18) du condensateur de mesure et du condensateur auxiliaire (17, 18) sont symétriques et de construction identique.

5. Système de mesure selon l'une des revendications 1 à 4
**caractérisé en ce que**
le condensateur auxiliaire (17, 18) est constitué de deux câbles de branchement se terminant à l'avant du condensateur de mesure et constitués et disposés de la même manière que les câbles d'alimentation (15, 16) du condensateur de mesure.

6. Système de mesure selon l'une des revendications 1 à 5,
**caractérisé en ce que**
le condensateur de mesure est formé d'une multiplicité de pistes conductrices plates, en particulier sous la forme d'un condensateur interdigital.

7. Système de mesure selon la revendication 6,
**caractérisé en ce que**
les pistes conductrices sont imprimées sur un support isolant (13) selon la technique de couche mince ou de couche épaisse.

8. Système de mesure selon l'une des revendications 1 à 7,
**caractérisé par**
un capteur de température (19) sous la forme d'une résistance à coefficient négatif de température, d'une résistance à coefficient positif de température ou d'un élément de température.

9. Système de mesure selon la revendication 8,
**caractérisé en ce que**
le capteur de température (19) est relié au capteur diélectrique (14) de manière à former une unité constructive.

10. Système de mesure selon la revendication 8 ou 9,
**caractérisé en ce que**
les câbles d'alimentation du capteur de température sont mis en place sur le support isolant (13) sous la forme de pistes conductrices.
